# EUROPEAN PATENT APPLICATION

(11) **EP 2 450 014 A1**
(43) Date of publication of application: **09.05.2012**
(21) Application number: 10794106.4
(22) Date of filing: 28.06.2010
(51) Int. Cl.: A61F 13/496

(54) **PANTS TYPE ABSORPTIVE ARTICLE**

(30) Priority: 01.07.2009 JP 2009157274
(71) Applicant: Kao Corporation, Chuo-Ku Tokyo 103-8210 (JP)
(72) Inventor: KOBAYASHI, Kenji, Haga-gun Tochigi 321-3497 (JP); ANDO, Kenji, Haga-gun Tochigi 321-3497 (JP); YANASHIMA, Takuo, Haga-gun Tochigi 321-3497 (JP); MORITA, Akio, Haga-gun Tochigi 321-3497 (JP)
(74) Representative: Vossius & Partner
(86) International application number: PCT/JP2010/060977
(87) International publication number: WO 2011/001937

(57) **Abstract**

A pull-on absorbent article 1 includes an outer cover 2 and an absorbent assembly 3 fixed on an inner side of the outer cover 2. The outer cover 2 has a front portion 2A to be worn around a wearer's front, a crotch portion 2C to be worn around a wearer's crotch, and a rear portion 2B to be worn around a wearer's rear. Opposite front side edges 2a, 2a and opposite rear side edges 2b, 2b of the outer cover are joined together to form a pair of side seals 4, 4. The outer cover 2 has non-bonded edges 2a" and 2b" located along the front and rear side edges 2a and 2b. The front side edge 2a and the rear side edge 2b are not joined to each other at the non-bonded edges 2a" and 2b". The non-bonded edges 2a" and 2b" are located closer to the crotch portion than a part of the front and rear side edges 2a and 2b where the side seals 4 are formed.

## Description

### Technical Field

The present invention relates to a pull-on (pants type) absorbent article, such as a pull-on disposable diaper.

### Background Art

A pull-on absorbent article is known, which includes an hourglass-shaped outer cover having a front portion to be worn around the wearer's front, a crotch portion to be worn around the wearer's crotch, and a rear portion to be worn around the wearer's rear and an absorbent assembly fixed to the inner side of the outer cover, with both the lateral side edges of the outer cover in the front portion and those in the rear portion being joined together to form a waist opening and a pair of leg openings (cf. patent documents 1 to 4 below).

### Citation List

### Patent Literature

Patent document 1: U.S. Patent 5836931
Patent document 2: JP 2005-279077A
Patent document 3: U.S. 2009/0281512A1
Patent document 4: JP 2003-88555A

### Summary of Invention

### Technical Problem

In the above described type of conventional pull-on absorbent articles, the lateral side edges of the outer cover in the front portion and those in the rear portion are joined together over the whole length of the respective sides.
Such pull-on absorbent articles provide a contouring fit about the leg by providing an elastic member of thread or band form along the leg openings, making the outer cover per se of an elastic sheet, or like means. Under these circumstances, attempts to further improve the fit can be accompanied by inconveniences due to strong constrictive forces around the wearer's legs or waist, such as red marks left on the skin or tightness exerted on the body of a wearer.

### Solution to Problem

The present invention provides a pull-on absorbent article which is of the type including an outer cover and an absorbent assembly fixed to an inner side of the outer cover. The outer cover has a front portion adapted to be worn around a wearer's front, a crotch portion adapted to be worn around a wearer's crotch, and a rear portion adapted to be worn around a wearer's rear. Lateral side edges of the front portion and lateral side edges of the rear portion are joined together to form a pair of opposite side seals. The outer cover has a non-bonded edge located along each of the lateral side edges of each of the front and rear portions. The front and rear portions are not joined to each other at the non-bonded edge. The non-bonded edge is located closer to the crotch portion than a. part of each of the lateral side edges of each of the front and rear portions where the side seal is formed.

### Advantageous Effects of Invention

The pull-on absorbent article of the invention provides a good fit around the legs of a wearer without excessively constricting the legs and other body parts of the wearer and is yet easy to put on, providing ease for a user to insert wearer's legs through the leg openings.

### Brief Description of Drawings

[Fig. 1] Fig. 1 is a side view of a disposable pull-on diaper according to an embodiment of the invention while in use (while worn).
[Fig. 2] Fig. 2 is a plan of the disposable pull-on diaper shown in Fig. 1 in its flat-out, uncontracted state with a part cut away. As used herein, the term "flat-out, uncontracted state" means a state in which a pull-on absorbent article is opened by tearing the side seals apart and with every elastic member straightened up to its design dimension (the dimension of an article in a flat-out configuration with any influences of elastic members eliminated).
[Fig. 3] Fig. 3 is an enlarged fragmentary view of the front or the rear portion of the outer cover of the disposable pull-on diaper shown in Fig. 1, with a part cut away.
[Fig. 4] Fig. 4(a) and Fig. 4(b) each show a lateral cross-section of an elasticized extension portion of the disposable pull-on diapers shown in Fig. 1, of which Fig. 4(a) represents a state in which an elastic member 24 is straightened up to unfold the elasticized extension portion, and Fig. 4(b) represents a state in which the elastic member 24 contracts to form folds 29.
[Fig. 5] Fig. 5 is a view of a disposable pull-on diaper according to another embodiment of the invention (equivalent to Fig. 2).

### Description of Embodiments

The invention relates to a pull-on absorbent article providing a good fit around the legs of a wearer without excessively constricting the legs and other body parts of the wearer.

The pull-on absorbent article of the invention will be described based on its preferred embodiments while referring to the accompanying drawings.
A disposable pull-on diaper 1 according to the first embodiment of the invention (hereinafter simply referred to as the diaper 1) includes an outer cover 2 and an absorbent assembly 3 fixed to an inner side of the outer cover. The outer cover 2 has a front portion 2A to be worn around a wear's front, a crotch portion 2C to be worn around a wear's crotch, and a rear portion 2B to be worn around a wear's rear. Front side edges 2a and 2a of the front portion 2A (hereinafter referred to as front side edges) and rear side edges 2b and 2b of the rear portion 2B (hereinafter referred to as rear side edges)are joined together to form a pair of opposite side seals 4 and 4.
The outer cover 2 has non-bonded edges 2a" and 2b" along each front side edge and each rear side edge, respectively. The front portion and the rear portion are not joined to each other at the non-bonded edges 2a" and 2b". The non-bonded edges 2a" and 2b" are located closer to the crotch portion than the part of each front side edge and each rear side edge where the side seal is formed. The part of the side edges where the side seal is formed will hereinafter be referred to as a bonded side edge.

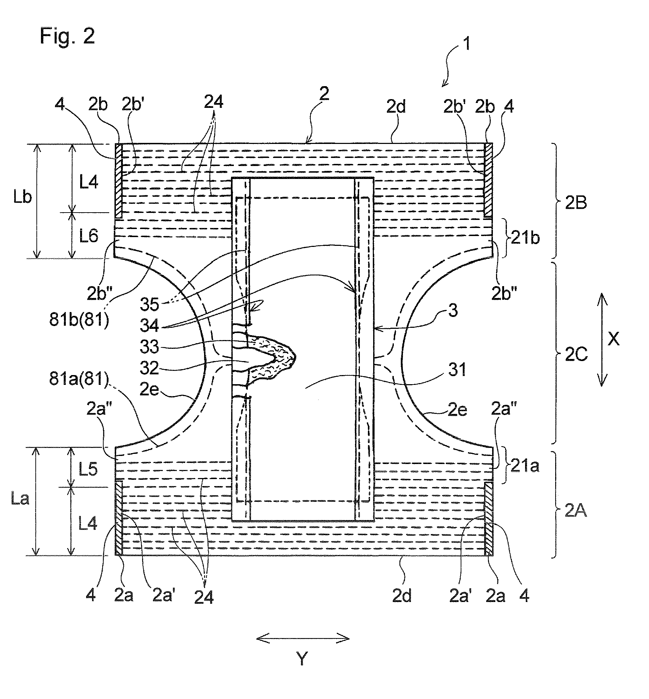

The diaper 1 will be illustrated in more detail. As illustrated in Fig. 2, the absorbent assembly 3 of the diaper 1 includes a liquid permeable topsheet 31, a liquid impermeable or water repellent backsheet 32, and a liquid retentive absorbent core 33 interposed between the two sheets 31 and 32. The absorbent assembly 3 has a rectangular shape longer in the longitudinal direction of the diaper 1 (absorbent article). The term "longitudinal direction" of the diaper 1 refers to a direction extending from the front portion A through the crotch portion C to the rear portion B or vice versa with the diaper 1 in a flat-out, uncontracted state (direction X in Fig. 2), and the term "lateral direction" of the diaper 1 refers to a direction perpendicular to the longitudinal direction (direction Y in Fig. 2).

A pair of side cuffs 34 and 34 formed of a liquid resistant or water repellent and breathable material are disposed one along each lateral side edge of the absorbent assembly 3. The side cuff 34 has an elastic member 35 disposed along its free edge in a stretched state. While the diaper 1 is worn, the elastic member 35 contracts to raise the side cuff 34 to block a lateral flow of a body fluid out of the absorbent assembly 3. The topsheet 31, backsheet 32, and absorbent core 33 may be made of any material commonly used in this type of absorbent articles.

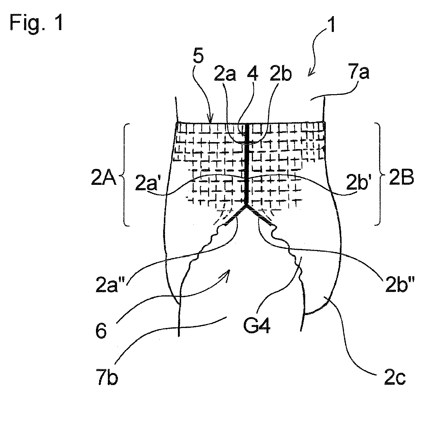

The outer cover 2 has its longitudinal middle part narrowed to have an hourglass shape in a plan view when the diaper 1 is in a flat-out, uncontracted state (see Fig. 2). The front portion 2A of the outer cover 2 has a pair of opposite front side edges 2a, 2a extending in the diaper longitudinal direction (direction X) and a longitudinal end edge 2d extending in the diaper lateral direction (direction Y). Similarly, the rear portion 2B of the outer cover 2 has a pair of opposite rear side edges 2b, 2b extending in the diaper longitudinal direction (direction X) and a longitudinal send edge 2d extending in the diaper lateral direction (direction Y). The crotch portion 2C of the outer cover 2 has a pair of opposite, inwardly concave side edges 2e, 2e. The concave side edges 2e, 2e illustrated are arc-shaped.
The parts 2a', 2a' of the front side edges 2a, 2a of the outer cover 2 and the respectively facing parts 2b', 2b' of the rear side edges 2b, 2b of the outer cover 2 are bonded together, whereby the diaper 1 has, as illustrated in Fig. 1, side seals (only one of which is shown), a waist opening 5, and a pair of leg openings 6,6 (only one of which is shown). The bonding of the side edges is performed by, for example, heat sealing, high frequency sealing, ultrasonic sealing, or adhesive application. In Fig. 1 is shown the waist opening 5 and the leg opening 6 through which the torso 7a and leg 7b of a child are put, respectively. The diaper 1 of the present embodiment is symmetric about a longitudinal centerline (not shown) extending in the diaper longitudinal direction (direction X).

The absorbent assembly 3 is fixed with an adhesive to the longitudinal (direction X) and lateral (direction Y) middle of the outer cover 2. One of the longitudinal end portions of the absorbent assembly 3 (the portion overlapping the front portions of the outer cover 2) is fixed to the lateral middle region of the front portion 2A, while the other (the portion overlapping the rear portion 2B of the outer cover 2) is fixed to the lateral middle region of the rear portion 2B.

In the cases where the front and the rear portion 2A, 2B of the outer cover 2 are formed of sheet materials that gather on contraction of elastic members 24 having been secured in their stretched state, the opposite front side edges 2a and 2a are preferably parallel with each other and so are the opposite rear side edges 2b and 2b, with the elastic members 24 in the stretched state as illustrated in Fig. 2. In the cases where extensibility and contractibility are exerted by elastic filaments and the like having been secured in their substantially unstretched state to extensible nonwoven fabric as in a fifth embodiment of the invention hereinafter described, the opposite front side edges 2a and 2a are parallel with each other and so are the opposite rear side edges 2b and 2b, with the nonwoven fabric being in its substantially non-stretched state.
Nevertheless, the opposite front side edges 2a and 2a do not need to be parallel, and so do the opposite rear side edges 2b and 2b. For example, the distance between the opposite side edges may increase or decrease from the end edge 2d toward the crotch portion 2C in the front or the rear portion 2A or 2B, or may once increase and then decrease or once decrease and then increase in that direction.

As illustrated in Fig. 2, the length of the side seal 4 is smaller than either the length La of the front side edge 2a or the length Lb of the rear side edge 2b. That is, the outer cover 2 has non-bonded edges 2a" and 2b" as a part of the front side edge 2a and as a part of the rear side edge 2b, respectively, where the front portion 2A and the rear portion 2B are not bonded to each other.
As illustrated in Figs. 1 and 2, the front and the rear side edges 2a and 2b include the parts 2a' and 2b' along which the front side edge 2a and the rear side edge 2b are bonded to each other to form the side seal 4. The non-bonded edges 2a" and 2b" are located closer to the crotch portion C than the parts 2a' and 2b' of the front and the rear side edges 2a and 2b, respectively, where the side seal 4 is formed.

The outer cover 2 of the diaper 1 has a portion 21 a located between the opposing non-bonded edges 2a", 2a" of the front section 2A. The portion 21a has extensibility in the diaper lateral direction (direction Y). Also, the outer cover 2 has a portion 21b located between the opposing non-bonded edges 2b", 2b" of the rear section 2B. The portion 21b has extensibility in the diaper lateral direction (direction Y). As used herein, the phrase "to have extensibility in the lateral direction of an absorbent article" is intended to mean that the extensibility is exhibited only in the lateral direction (direction Y), in both the lateral direction (direction Y) and the longitudinal direction (direction X), or in any planar direction.
The portion 21a between the opposing non-bonded edges 2a", 2a" in the front section 2A and the portion 21b between the opposing non-bonded edges 2b", 2b" in the rear section 2B will be called "extension portions 21a and 21b", respectively in the sense that these portions are extensions that extend downward (toward the crotch portion) from the region between the opposite side seals 4,4.

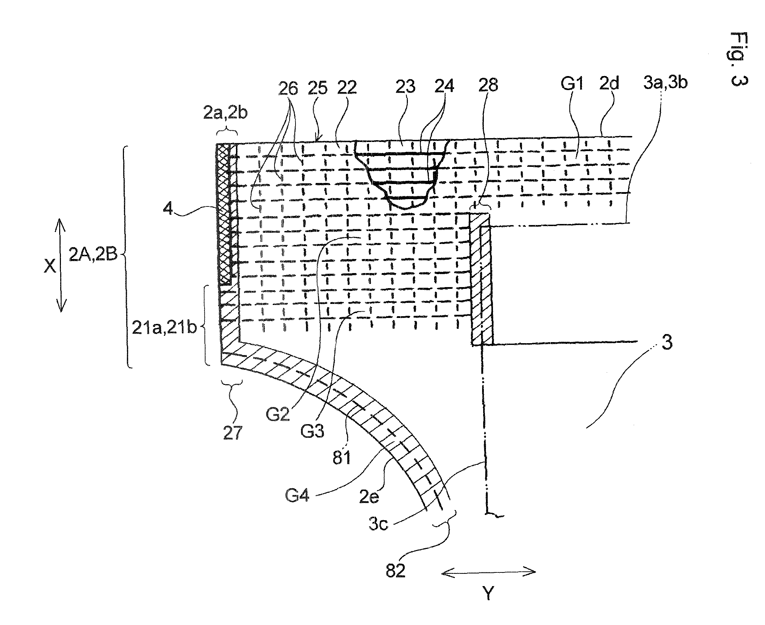

As illustrated in Fig. 3, the outer cover 2 of the diaper 1 includes an outer sheet 22, an inner sheet 23 laid on the inner side of the outer sheet 22, and elastic members 24 interposed between the two sheets 22 and 23. Each of the outer sheet 22 and the inner sheet 23 is formed of a single sheet continuous from the front portion 2A through the crotch portion 2C to the rear portion 2B.

As illustrated in Fig. 3, the front and the rear portion 2A and 2B of the outer cover 2 both have a structure in which a plurality of elastic members 24 of thread form are disposed between the outer sheet 22 defining the exterior of the diaper and the inner sheet 23 laid on the inner side of the outer sheet 22 so that the front and the rear portion 2A and 2B are each provided with an elasticized waist region G1, an elasticized below-waist region G2, and an elasticized extension region G3.
The elasticized waist region G1 is outward from the respective longitudinal end 3a or 3b of the absorbent assembly 3 in the diaper longitudinal direction (direction X). The elasticized extension region G3 is formed in each of the extension portion 21a of the front outer cover 2A and the extension portions 21b of the rear portion 2B. The elasticized below-waist region G2 is between the elasticized waist region G1 and the elasticized extension region G3 along direction X in each of the front and the rear portion 2A and 2B. Both the elasticized below-waist region G2 and the elasticized extension region G3 are laterally outward from each lateral side edge of the absorbent assembly 3. That is, the elasticized below-waist region G2 and the elasticized extension region G3 show extensibility in at least the regions located laterally outward from each lateral side edge of the absorbent assembly 3 in each of the front and rear portions 2A and 2B. On the other hand, it is preferred that the elastic members 24 that have been once disposed in the diaper manufacturing be cut in a region overlapping the absorbent assembly, particularly a region overlapping the middle part of the absorbent assembly 3 so that the region may be de-elasticized. The position where the absorbent assembly 3 is placed is indicated by the long dashed double-dotted line in Fig. 3.

The outer and the inner sheets 22 and 23 are bonded to each other at a large number of discretely arranged bonds 26 in the elasticized waist region G1, elasticized below-waist region G2, and elasticized extension region G3. Specifically, bonds 26 are discretely arranged in lines extending in the diaper longitudinal direction (direction X), and the lines of bonds are spacedly disposed in the diaper lateral direction (direction Y). The positions in direction X of the bonds of a line coincide with those of the bonds of adjacent lines.

A plurality of elastic members 24 in each of the elasticized regions G1 to G3 are arranged such that each one of them passes between bonds 26 adjacent in the longitudinal direction. Each elastic member 24 is not fixed to either the outer sheet 22 or the inner sheet 23 at other than the fixing parts hereinafter described (an end fixing part 27 or an absorbent assembly-side fixing part 28).

The elasticized waist region G1 in the front portion 2A has, along the front side edges 2a and 2a, an opposite pair of end fixing parts 27 in which the outer and the inner sheet 22 and 23 are bonded to each other with an adhesive (see Fig. 3). Each of the elastic members 24 disposed in the elasticized waist region G1 is secured between the sheets 22 and 23 at the opposite pair of the end fixing parts 27, while not being fixed to either the sheet 22 or the sheet 23 between the opposite end fixing parts 27. The same configuration applies to the elasticized waist region G1 in the rear portion 2B.
The elasticized below-waist region G2 and the elasticized extension region G3 of the front portion 2A each have an end fixing part 27 along one side edge of the front portion 2A where the outer sheet 22 and the inner sheet 23 are bonded with an adhesive and an absorbent assembly-side fixing part 28 near the side edge 3c of the absorbent assembly 3 where the outer sheet 22 and the inner sheet 23 are bonded with an adhesive. Each of the elastic members 24 disposed in the elasticized below-waist region G2 and the elasticized extension region G3 is secured between the sheets 22 and 23 at the end fixing part 27 and at the absorbent assembly-side fixing part 28, while not being fixed to either the sheet 22 or the sheet 23 between the end fixing part 27 and the absorbent assembly-side fixing part 28. The same configuration applies to the elasticized below-waist region G2 and the elasticized extension region G3 in the rear portion 2B.

The absorbent assembly-side fixing part 28 may be formed to straddle the side edge 3c of the absorbent assembly 3 as is depicted in Fig. 3 or may be formed to entirely overlap the absorbent assembly 3. When the fixing part 28 entirely overlaps the absorbent assembly 3, the outboard edge of the absorbent assembly-side fixing part 28 may be coincident with the side edge 3c of the absorbent assembly 3 or inboard of the side edge 3c of the absorbent assembly 3 at a prescribed distance.
Otherwise, the absorbent assembly-side fixing part 28 may be formed outboard of the side edge 3c of the absorbent assembly 3.
Fig. 3 presents a view of the diaper 1 from the side of the outer sheet 22. The absorbent assembly 3 depicted in Fig. 3 is fixed to the inner sheet 23.

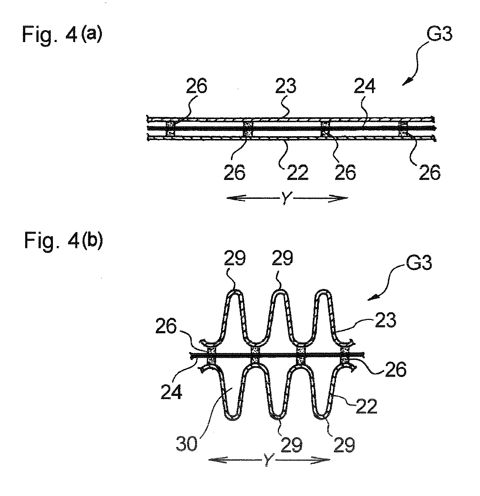

On contraction of the elastic members 24 in the elasticized waist region G1, elasticized below-waist region G2, and elasticized extension region G3, each of the sheets 22 and 23 is deformed to bulge outward to form folds 29 between every pair of adjacent lines of bonds. At the same time, vacant spaces 30 are formed between the sheets 22 and 23, being defined by every fold 29 of the sheet 22 and every fold 29 of the sheet 23 (see Fig. 4). The folds 29 and the vacant spaces 30 provide improved softness of the front portion 2A and the rear portion 2B, which leads to improved feel to the touch. Furthermore, air flows easily through the valleys between the folds 29 on the skin-facing side to provide good protection against skin overhydration. When, in particular, the inner sheet 23 is formed of an air-permeable sheet, like nonwoven fabric, the humid air inside the diaper 1 escapes outside more easily.

The outer cover 2 also has a leg elastic member 81 fixed in its stretched state along each concave edge 2e of the crotch portion 2C. On contraction of the leg elastic member 81, a leg gather G4 forms along the concave edge 2e. The leg elastic member 81 includes a first elastic member 81a disposed from the front side edge 2a to near the center of the crotch portion 2C in direction X and a second elastic member 81b disposed from the rear side edge 2b to near the center of the crotch portion 2C in direction X (see Fig. 2). The leg elastic member 81 is disposed between the outer sheet 22 and the inner sheet 23 and fixed to the sheets 22 and 23 in its stretched state in a concave fixing part 82 (see Fig. 4) where the sheets 22 and 23 are bonded to each other via an adhesive.
The leg elastic member 81 composed of the first and the second elastic member 81 a and 81b may be replaced with a single continuous elastic member extending from the front portion 2A through the crotch portion 2C to the rear portion 2B. The leg elastic member 81 may be a plurality of elastic members on each lateral side of the diaper 1.

Since the diaper 1 of the first embodiment has the non-bonded edges 2a" and 2b" along each front side edge 2a and each rear side edge 2b, respectively, on putting the diaper 1 on a wearer, the non-bonded edge 2a" of the front portion 2A and the non-bonded edge 2b" of the rear portion 2B separate from each other toward the front and the rear in the shape of an inverted letter Y As a result, the wearer's thigh is not completely encircled by the leg elastic member(s), and the non-bonded edges 2a" and 2b" contract without being excessively pressed against the thigh. The constrictive force exerted onto the thigh is thus lessened while providing a good fit around the leg circumference.
Furthermore, by virtue of the presence of the non-bonded edges 2a" and 2b", the contractive force over the distance between the opposite non-bonded edges (between 2a" and 2a'' and between 2b" and 2b") in the front portion 2A or the rear portion 2B unexpectedly functions to ensure the contractive force of the elastic members 24 existing between the opposite bonded side edges (between 2a' and 2a' and between 2b' and 2b') in the waist elasticized region G1 and the below-waist elasticized region G2. This leads to a good fit to a wearer and prevention of sliding down of the absorbent article during wear.

The presence of the non-bonded edges 2a" and 2b" in the front and the rear side edges 2a and 2b, respectively, also offers the advantage that the side seal 4 is easier to tear apart in exchanging diapers. Since the non-bonded edges 2a" and 2b" are capable of separating apart from each other, another advantage is that it is easy to get the wearer's legs through the leg openings. That is, the diaper is easy to apply.

The contractibility of the extension portions 21a and 21b in the diaper lateral direction is preferably such that the length, in the diaper lateral direction, of the portion laterally outward of the side edge 3c of the absorbent assembly 3 in each of the extension portions 21a and 21b with the diaper 1 in a contracted state is 15% to 90%, more preferably 20% to 60%, even more preferably 25% to 50%, relative to the length of the same portion with the diaper 1 in a flat-out, uncontracted state.

To further ensure one or more of the aforementioned effects, it is preferred that the non-bonded edges 2a" and 2b" of the front portion 2A and the rear portion 2B, respectively, have respective extension lengths L5 and L6 (the length extending from the side seal 4 toward the crotch portion 2C) ranging from 5% to 150%, more preferably 25% to 70%, of the length L4 of the side seal 4.
For the same purpose, it is preferred that the length L5 of the non-bonded edge 2a" in the front portion 2A be in the range of from 5% to 60%, more preferably 20% to 40%, of the length La of the front side edge 2a and that the length L6 of the non-bonded edge 2b" in the rear portion 2B be in the range of from 5% to 60%, more preferably 20% to 40%, of the length Lb of the rear side edge 2b.
The extension lengths L5 and L6 of the non-bonded edges 2a" and 2b", respectively, are preferably 10 mm or more, more preferably 20 mm or more. For use by children, the extension lengths L5 and L6 of the non-bonded regions 2a" and 2b", respectively, are preferably 10 to 100 mm, more preferably 20 to 70 mm. For use by adults, the extension lengths L5 and L6 of the non-bonded edges 2a" and 2b", respectively, are preferably 10 to 150 mm, more preferably 20 to 100 mm.

From the viewpoint of easing the insecurity feeling about leakage by reducing the skin exposure observed from the wearer's front, it is preferred that the extension length L5 of the non-bonded edge 2a" located at the front portion 2A and the extension length L6 of the non-bonded edge 2b" located at the rear portion 2B be substantially equal. As used herein, the expression "substantially equal" is intended to mean that the difference in length between L5 of the non-bonded edge 2a" in the front portion 2A and L6 of the non-bonded edge 2b" in the rear portion 2B is within 5 mm.
The front and the rear portion 2A and 2B having the respective extension portions 21a and 21b, the extension length L6 of the extension portion 21b in the rear portion 2B may be greater than the extension length L5 of the extension portion 21a in the front portion 2A. In this case, the extension length L6 is preferably 1.1 to 2.5 times the extension length L5.
In view of improvement on fit and appearance of the rear side, it is also a preferred embodiment for the pull-on absorbent article of which the extension portion 21b in the rear portion 2B is longer than the extension portion 21a of the front portion 2A to have extensibility in the extension portion 21b of the rear portion 2B but no extensibility in the extension portion 21a of the front portion 2A.

The outer sheet 21 and the inner sheet 22 may be of any sheet material conventionally used in this type of articles but are preferably formed of nonwoven fabric. In view of softness and the like, a single sheet or a laminate of two or more sheets of nonwoven fabric, such as air-through nonwoven, heat-rolled nonwoven, hydroentangled nonwoven, spun bonded nonwoven, and melt blown nonwoven fabrics, is particularly preferred. A nonwoven fabric integrally laminated with film is also useful. The molding material of the elastic members 24 is not particularly limited and may be any of known elastic materials of various kinds that have been used in absorbent articles, such as disposable diapers and sanitary napkins. Examples of such elastic materials include synthetic rubbers, such as styrene-butadiene, butadiene, isoprene, and neoprene, natural rubber, EVA, stretch polyolefins, and polyurethane. Forms of the elastic members preferably include a thread with a rectangular, square, circular or polygonal section, tape, or multifilamentous yarn.

The disposable pull-on diaper 1 of the first embodiment can efficiently be manufactured in a continuous manner, for example, as follows.
An adhesive for the formation of the end fixing parts 27, the absorbent assembly-side fixing parts 28 and the concave fixing parts 82 is applied to a wide continuous sheet as a precursor of the outer sheet 22 and a wide continuous sheet as a precursor of the inner sheet 23. The two sheets are then superposed on each other with elastic members 24 and the leg elastic members 81 inserted therebetween in their stretched state. The superposed two continuous sheets are introduced between a pressure roller having, on its peripheral surface, projections for forming bonds 26 and a facing anvil roller to be pressed in parts with heat thereby to form a large number of the bonds 26. The resulting laminate is pressed between a roller having projections and a smooth roller so that the elastic members 24 disposed between the absorbent assembly-side fixing parts 28 are cut to provide non-elasticized regions. Absorbent assemblies 3 are spacedly disposed to straddle the non-elasticized regions of the resulting continuous-form outer cover. Leg holes are cut out of the continuous-form outer cover either before or after the placement of the absorbent assembly 3 such that one leg hole defines facing concave side edges of adjacent diapers.

The continuous-form outer cover is then folded in two, followed by forming side seals 4 and 4 by heat sealing, ultrasonic sealing, high frequency sealing, or the like means. After the formation of the side seals 4 and 4, the continuous-form assembly is cut to size using a known cutting means (not shown) to obtain individual disposable pull-on diapers 1 having the aforementioned structure. The side seals 4 and 4 should be formed not to extend over the whole length of from one side edge of the continuous outer cover to the leg hole so that the diaper 1 may have the above-described non-bonded edges 2a" and 2b" along the lateral side edges of the front and the rear portions 2A and 2B. The side seals 4 and 4 are preferably formed using a sealing unit (not shown) having a pair of sealing rollers.
In carrying out the method of the present embodiment, the undescribed details are the same as those in conventional production of disposable pull-on diapers in what we call a lateral feed system.

A second embodiment of the invention will be described. Description of the disposable pull-on diapers of the second embodiment will generally be confined to the differences from the first embodiment, with the details in common omitted. The description of the first embodiment applies to the second embodiment with the exceptions noted hereunder. The elements or members of the second and the third embodiment common to the first embodiment are identified with the same reference numerals as in the first embodiment.

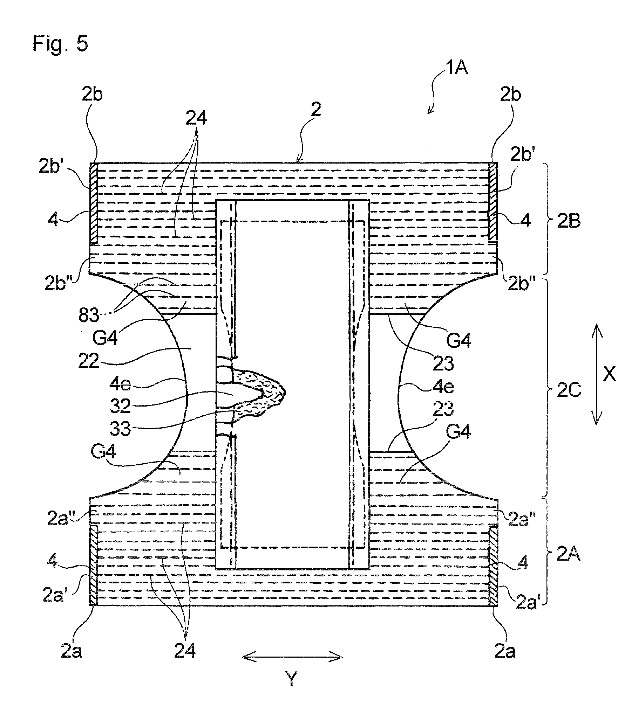

In the disposable pull-on diaper 1A of the second embodiment, as illustrated in Fig. 5, the outer cover 2 is formed of an outer sheet 22 that is a single sheet continuous from the front portion 2A through the crotch portion 2C to the rear portion 2B and two inner sheets 23: one in the front portion 2A and the other in the rear portion 2B.
The outer cover 2 has an elasticized crotch region G4 laterally outward from each side edge of the absorbent assembly 3 in the crotch portion C. The elasticized crotch region G4 has the same structure as the elasticized extension region G3 of the diaper 1 according to the first embodiment. That is, the elasticized crotch region G4 has a large number of bonds (not shown) at which the outer sheet 22 and the respective inner sheet 23 are bonded in the same pattern as of the bonds 26 in the elasticized extension region G3 of the diaper 1 of the first embodiment. The elasticized crotch region G4 has a plurality of elastic members 83 each passing between bonds 26 adjacent in the longitudinal direction. Similarly to the elasticized regions G1 to G3 of the diaper 1 of the first embodiment, each of the sheets 22 and 23 in the elasticized crotch region G4 of the diaper 1A of the second embodiment creates folds 29 and vacant spaces 30 enclosed by opposing folds 29 and 29 upon contraction of the elastic members 83 (see Fig. 4).

The elastic members 83 of the elasticized crotch region G4 are each secured between the sheet 22 and the facing sheet 23 with an adhesive, at one end thereof, at the absorbent assembly-side fixing part 28 similarly to the elasticized extension region G3 and, at the other end, at a fixing part provided along the concave side edge 4e. The elastic members 83 are not fixed to either the sheet 22 or 23 between the opposite fixing parts. The elasticized crotch region G4 which is located adjacent to the front portion 2A and the elasticized crotch region G4 which is located adjacent to the rear portion 2B are formed with the same geometry in the same manner. The disposable pull-on diaper 1A of the second embodiment has no leg elastic members 81 disposed along the concave edges 2e.

In the diaper 1A of the second embodiment, too, the front and the rear side edge 2a and 2b include the parts 2a' and 2b', respectively, along which the side seal 4 is formed and the non-bonded edges 2a" and 2b", respectively, where the front side edge and the rear side edge are not bonded to each other, and the elasticized extension region G3 exhibits extensibility. Thus, the diaper of the second embodiment produces the same effects as by the diaper 1 of the first embodiment.

Additionally, since, in the diaper 1A of the second embodiment, the folds 29 and the vacant spaces 30 as illustrated in Fig. 4 are also formed in the elasticized crotch portion G4. As a result, the softness and the feel to the touch are improved, and the humid air inside the diaper escapes outside more easily.

The third to fifth embodiments of the invention will then be described. Description of the disposable pull-on diapers of the third and fourth embodiments will generally be confined to the differences from the first and the second embodiment, with the details in common omitted. The description of the first and second embodiments applies to the third and fourth embodiments with the exceptions noted hereafter. The elements or members of the third and fourth embodiments common to the first and second embodiments are identified with the same reference numerals as in the first and second embodiments.

The disposable pull-on diaper of the third embodiment exhibits extensibility in the same geometry as in the first embodiment, except that each of the elasticized waist region G1, the elasticized below-waist region G2, and the elasticized extension region G3 in each of the front portion 2A and the rear portion 2B has a structure composed of an outer sheet 22 that defines the exterior of the diaper and an inner sheet 23 on the inner side of the outer sheet 22 bonded to each other over their entire area with elastic members 24 secured in their stretched state between the two sheets 22 and 23 over the entire area of the respective elasticized regions.
In the diaper of the third embodiment, each of the elasticized waist region G1, the elasticized below-waist region G2, and the elasticized extension region G3 forms irregular creases or folds by the contraction of the elastic members 24 but does not form vacant spaces 30 as formed in the first embodiment.

The disposable pull-on diaper of the fourth embodiment exhibits extensibility in the same geometry as in the second embodiment, except that each of the elasticized waist region G1, the elasticized below-waist region G2, and the elasticized extension region G3 in the front portion 2A and the rear portion 2B and the elasticized crotch region G4 in the crotch portion 2C has a structure composed of an outer sheet 22 that defines the exterior of the diaper and an inner sheet 23 on the inner side of the outer sheet 22 bonded to each other over their entire area with elastic members 24 secured in their stretched state between the two sheets 22 and 23 over the entire area of the respective elasticized regions.
In the diaper of the fourth embodiment, the elasticized waist region G1, the elasticized below-waist region G2, the elasticized extension region G3, and the elasticized crotch region G4 form irregular creases or folds by the contraction of the elastic members 24 but do not form vacant spaces 30 as formed in the first embodiment.

In the third and fourth embodiments, the front side edges 2a and the rear side edges 2b have the parts 2a' 2b", respectively, where the side seal 4 is formed and the non-bonded edges 2a" and 2b", respectively, where the front side edge and the rear side edge are not bonded together, and the elasticized extension region G3 exhibits extensibility. Thus, the diapers of the third and fourth embodiments produce the same effects as by the diaper 1 of the first embodiment.

In the disposable pull-on diaper of the fifth embodiment, the outer cover 2 includes an inner sheet that is a single sheet continuous from the front portion 2A through the crotch portion 2C to the rear portion 2B and two outer sheets: one in the front portion 2A and the other in the rear portion 2B. The two outer sheets are fixed to the inner sheet on their peripheries with an adhesive.
The inner sheet or the outer sheets is/are formed of an extensible sheet obtained by fixing a number of elastic filaments in a parallel configuration to extensible nonwoven fabric in their substantially unstretched state over their whole length, thereby to elasticize the diaper in the lateral direction.
Such an extensible sheet is exemplified by the one described in US 2010/0075103A, which is incorporated herein by reference in its entirety. The extensible sheet is preferably composed of two sheets of nonwoven fabric, which may be the same or different, and the elastic filaments therebetween. The elastic filaments may be synthetic or natural rubber threads or filaments obtained by dry spinning (e.g., melt spinning) or wet spinning. It is preferable that the elastic filaments obtained by melt spinning are directly fed onto the nonwoven fabric without being once taken-up or stocked. An extensible sheet in which elastic filaments, either continuous or staple, are randomly arranged may also be useful.

The extensible sheet for use in the fifth embodiment is preferably, for example, an extensible sheet composed of two sheets of nonwoven fabric: a first nonwoven fabric and a second nonwoven fabric and a number of elastic filaments sandwiched between the two sheets. The first and the second nonwoven fabric may be the same or different. As referred to above, when the two nonwoven fabrics are said to be "the same", this means that they are totally equal in all the attributes including production process, material, thickness and length of constituent fibers, thickness, and basis weight. Two nonwoven fabrics different in any one of these attributes are regarded to be different. Both the first and the second nonwoven fabric are extensible. The term "extensible" as used herein with respect to nonwoven fabrics is contemplated to include not only a nonwoven fabric whose constituent fibers per se are extensible but also a nonwoven fabric whose constituent fibers are not per se extensible but which shows extensibility as a whole as a result of debonding of constituent fibers that have been bonded at their intersections, structural change of a three-dimensional structure formed of a plurality of fibers bonded to one another, breaks of the constituent fibers, or straightening out of slack fibers. The nonwoven fabrics may have extensibility or be made extensible before the elastic filaments are bonded thereto (in the form of stock roll). Alternatively, the nonwoven fabrics may be inextensible before the elastic filaments are bonded thereto and, after being joined with the elastic filaments, be subjected to processing to be made extensible. Methods for making a nonwoven fabric extensible include heat treatment, stretching between spaced pairs of rollers, stretching by a bite between corrugating members (such as gears), and stretching by a tenter. It is preferred that the nonwoven fabrics not be extensible in the form of stock roll so that they may be conveyed stably during fusion bonding the elastic filaments to the nonwoven fabrics.

In the disposable pull-on diaper of the fifth embodiment, too, the front and the rear side edges 2a and 2b include the parts 2a' and 2b', respectively, along which the side seal 4 is formed and the non-bonded edges 2a" and 2b", respectively, where the front side edge and the rear side edge are not bonded to each other, and the extension regions 21a and 21 b in the front portion 2A and the rear portion 2B, respectively, exhibit extensibility in the diaper lateral direction. Thus, the diaper of the fifth embodiment produces the same effects as by the diaper of the first embodiment.

While the invention has been described with respect to the preferred embodiments thereof, it should be understood that the invention is not construed as being limited thereto.
For example, the extensible sheet used in the disposable pull-on diaper of the fifth embodiment may be used as an outer sheet and/or an inner sheet in a disposable pull-on diaper of the type in which each of the outer and the inner sheet making up the outer cover is a single sheet continuous from the front portion 2A through the crotch portion 2C to the rear portion 2B.

There may be a non-bonded region outboard of each side seal 4 formed along the side edges 2a and 2b of the outer cover.

The pull-on absorbent article of the invention may be a pull-on sanitary napkin and the like as well as a disposable pull-on diaper for children or adults.
The invention may be applied to pull-on garments having no absorbent assembly, such as disposable underwear.

## Claims

1. A disposable pull-on absorbent article comprising an outer cover and an absorbent assembly fixed to an inner side of the outer cover, the outer cover having a front portion adapted to be worn around a wearer's front, a crotch portion adapted to be worn around a wearer's crotch, and a rear portion adapted to be worn around a wearer's rear, lateral side edges of the front portion and lateral side edges of the rear portion being joined together to form a pair of opposite side seals, wherein
the outer cover has a non-bonded edge located along each of the lateral side edges of each of the front and rear portions;
the front and rear portions are not joined to each other at the non-bonded edge; and
the non-bonded edge is located closer to the crotch portion than a part of each of the lateral side edges of each of the front and rear portions where the side seal is formed.

2. The pull-on absorbent article according to claim 1, wherein the outer cover has a region located (between the non-bonded edges in the front portion and/or the rear portion, the region having extensibility in the lateral direction of the absorbent article.

3. The pull-on absorbent article according to claim 1 or 2, wherein the non-bonded edge has a length extending from the respective side seal toward the crotch portion of from 5% to 150% relative to the length of the side seal.

4. The pull-on absorbent article according to any one of claims 1 to 3, wherein the non-bonded edge located at the front portion and the non-bonded edge located at the rear portion extend from the respective side edge toward the crotch portion, and have substantially equal lengths.

5. The pull-on absorbent article according to any one of claims 1 to 4, wherein the outer cover comprises an outer sheet, an inner sheet located on the inner side of the outer sheet, and elastic members located between the outer sheet and the inner sheet, and
the outer sheet and/or the inner sheet comprise a single sheet continuous from the front portion through the crotch portion to the rear portion.

6. A pull-on garment comprising an outer cover having a front portion adapted to be worn around a wearer's front, a crotch portion adapted to be worn around a wearer's crotch, and a rear portion adapted to be worn around a wearer's rear, lateral side edges of the front portion and lateral side edges of the rear portion being joined together to form a pair of opposite side seals, wherein
the outer cover has a non-bonded edge located along each of the lateral side edges of each of the front and rear portions;
the front and rear portions are not joined to each other at the non-bonded edge_{;} and
the non-bonded edge is located closer to the crotch portion than a part of each of the lateral side edges of each of the front and rear portions where the side seal is formed.
